# EUROPEAN PATENT SPECIFICATION

(11) **EP 3 919 172 B1**
(45) Date of publication and mention of the grant of the patent: **29.01.2025**
(21) Application number: 21170205.5
(22) Date of filing: 23.04.2021
(51) Int. Cl.: C12M 3/00, C12M 3/06, B01L 9/00, B01L 3/00, C12M 1/00

(54) **MICROFLUIDIC DEVICE**
MIKROFLUIDISCHE VORRICHTUNG
DISPOSITIF MICROFLUIDIQUE

(30) Priority: 04.06.2020 JP 2020097948
(43) Date of publication of application: 08.12.2021
(73) Proprietor: Ushio Denki Kabushiki Kaisha, Tokyo-to 100-8150 (JP)
(72) Inventor: YAMANAKA, Makoto, Tokyo, 100-8150 (JP)
(74) Representative: Maiwald GmbH

(56) References cited:
- WO-A1-2020/066609
- US-A1- 2009 246 087
- US-A1- 2016 003 859
- US-A1- 2018 327 700

## Description

### TECHNICAL FIELD

The present invention relates to a microfluidic device.

### BACKGROUND ART

Conventionally, culture dishes and plates have been used to cultivate cells and tissues for analysis and other purposes. Cultivation of cells and tissues using culture dishes or culture plates is conducted in a two-dimensional (flat) environment, thus failing to reproduce the extracellular microenvironment (for example, US 20160003859 A1 and US 20090246087 A1). In recent years, microfluidic devices having microfluidic channels have been proposed (also called "cell culture chips," "biochips," "microchips," or "microfluidic chips"). The microfluidic channels are capable of achieving a three-dimensional (3D) cell culture and experimental environment, which has been difficult with conventional methods. Patent Document 1 and EP 3 858 974 A1 disclose examples of such a microfluidic device. A microfluidic device with the features of the preamble of present claim 1 is disclosed in US 2018/327700 A1.

Incidentally, in a case that part of liquid injected into a culture plate evaporates, the concentration of the liquid in the plate varies, resulting in a significant impact on the result of processing and analysis. Hence, there is a need to suppress the evaporation of the liquid injected into the culture plate and to reduce the variation in the concentration of the liquid. Patent Document 2 discloses a method of maintaining a high humidity in the atmosphere of wells of the culture plate by providing multiple slot-shaped reservoirs around the wells. This method allows to suppress the evaporation of liquid components injected into the culture plate in a conventional culture plate, which is not a microfluidic device.

### CITATION LIST

### PATENT DOCUMENT

Patent Document 1: JP-A-2018-047614
Patent Document 2: US 5587321 A
Patent Document 3: US 2018/327700 A1

### SUMMARY OF INVENTION

### Technical Problem

Even in microfluidic devices, it is desirable to reduce the concentration variation associated with evaporation of the liquid. According to the inventor's consideration, since the amount of liquid injected into the microfluidic device is smaller than the amount of liquid injected into a conventional culture plate such as disclosed in Patent Document 2, the variation in concentration of the liquid component caused by evaporation of a part of the liquid component injected into the microfluidic device is larger than the variation in liquid concentration of a conventional culture plate such as disclosed in Patent Document 1. Hence, the inventor has realized that evaporation suppression of liquid components in microfluidic devices needs to be more precisely controlled than that in conventional culture plates.

However, since the plate on which the microfluidic channel is formed is thinner and smaller than a conventional culture plate, even when a reservoir is provided as described in Patent Document 2 on the plate on which the microfluidic channel is formed, the reservoir does not store a sufficient amount of liquid, thus failing to suppress evaporation sufficiently.

It is an object of the present invention to provide a microfluidic device that can effectively suppress the evaporation of liquid injected into a microfluidic device.

### Solution to Problem

The microfluidic device of the present invention has the features described in claim 1 and includes a plate having a first main surface that has an opening area in which a plurality of openings for injecting fluid are formed, a second main surface being the opposite side of the first main surface, and an outer side surface communicating the first main surface and the second main surface; and a reservoir that has an opening with a bottom and is capable of storing liquid, the opening standing above the first main surface outside the opening area.

As is explained in detail below, the microfluidic device of the present invention has an opening in the reservoir with a bottom (an opening with a bottom) that is located higher than the first main surface of the plate, thus enabling the reservoir to be mounted deeper than the case where the reservoir is mounted inside a thin-structured plate. Hence, the reservoir in the present invention can store larger amount of liquid compared with a reservoir provided inside a plate. As a result, the reservoir can ensure a sufficient amount of liquid to maintain the atmosphere in the opening area at high humidity. Therefore, the evaporation of liquid components from the liquid injected into the opening can be effectively suppressed.

Furthermore, the microfluidic device is provided with a holder having an inner wall surface that is located outside the outer side surface of the plate and a support surface on which the second main surface of the plate is mounted.

The reservoir may include a bottom located outside the opening area of the plate, an inner frame standing upward from the bottom to above the first main surface and being separated inward from the inner wall surface of the holder, and an outer frame standing upward from the bottom between the inner wall surface and the inner frame.

The reservoir may be configured separately from the holder, and the bottom may be located above the first main surface. This configuration allows the bottom surface of the reservoir to be formed larger compared with a reservoir that is located on the same surface of the opening area, thus increasing the amount of liquid that can be stored. Therefore, the reservoir can ensure a sufficient amount of liquid to maintain the atmosphere in the opening area at high humidity.

The reservoir may be partitioned into a plurality of cells by a partitioning plate that communicates between the inner frame and the outer frame.

The total area of the inner bottom surface of the reservoir may be more than twice the total area of the openings in the plate.

The inner side surface of the reservoir may have a marker that serves as an indicator of the amount of replenishment fluid.

At least a portion of the reservoir may be provided with an evaporation enhancing member increasing the area of the gas-liquid surface.

The microfluidic device of the present invention may be provided with a cover that covers the opening area and the reservoir to form a gap between the cover and the upper end of the inner frame.

In the microfluidic device of the present invention, the plate may be constituted by bonding at least two substrates and include a space between the bonded substrates for being capable of culturing cells or tissues.

### ADVANTAGEOUS EFFECTS

This configuration provides a microfluidic device that can effectively suppress the evaporation of liquid components.

### BRIEF DESCRIPTION OF DRAWINGS

FIG. 1 is a schematic perspective view of a microfluidic device illustrating a first embodiment.
FIG. 2 is a schematic cross-sectional view of the microfluidic device taken along plane A1 in FIG. 1.
FIG. 3 is a schematic exploded perspective view of the microfluidic device illustrating the first embodiment.
FIG. 4 is a top view of a plate.
FIG. 5 is a schematic cross-sectional view of the plate taken along line A2 - A2 in FIG. 4.
Fig. 6 is a schematic cross-sectional view of a holder taken along plane A3 in FIG. 3.
FIG. 7 is a schematic cross-sectional view of the microfluidic device taken along plane A4 in FIG. 1.
FIG. 8 is a reference view of a microfluidic device without a partitioning plate.
FIG. 9 is an enlarged view of the A5 area in FIG. 3, with markers added.
FIG. 10 is a schematic perspective view of a microfluidic device illustrating a modified example.
FIG. 11 is a schematic cross-sectional view taken along plane A6 in FIG. 10.
FIG. 12 is a schematic cross-sectional view of a microfluidic device illustrating a second embodiment.

### DESCRIPTION OF EMBODIMENTS

The microfluidic device in accordance with the embodiments will now be described with reference to the drawings. It is noted that the following drawings are just schematically illustrated. In other words, the dimensional ratios on the drawings do not necessarily match the actual dimensional ratios, and the dimensional ratios between each drawing do not necessarily match either.

Hereinafter, the embodiments will be described with reference to the XYZ coordinate system as necessary. In the case of describing a direction to distinguish a positive direction from a negative direction, a positive or negative sign is added to the direction, such as "+X direction" or "-X direction". In the case of describing a direction without distinguishing a positive direction from a negative direction, the direction is simply expressed as "X direction". In other words, the term "X direction" used in this specification refers to both "+X direction" and "-X direction". The same manner applies to the Y direction and the Z direction. In the present embodiments, the horizontal plane is parallel to the XY plane, and the vertical direction is the -Z direction. The plate described below is illustrated to be parallel to the XY plane.

### < First Embodiment>

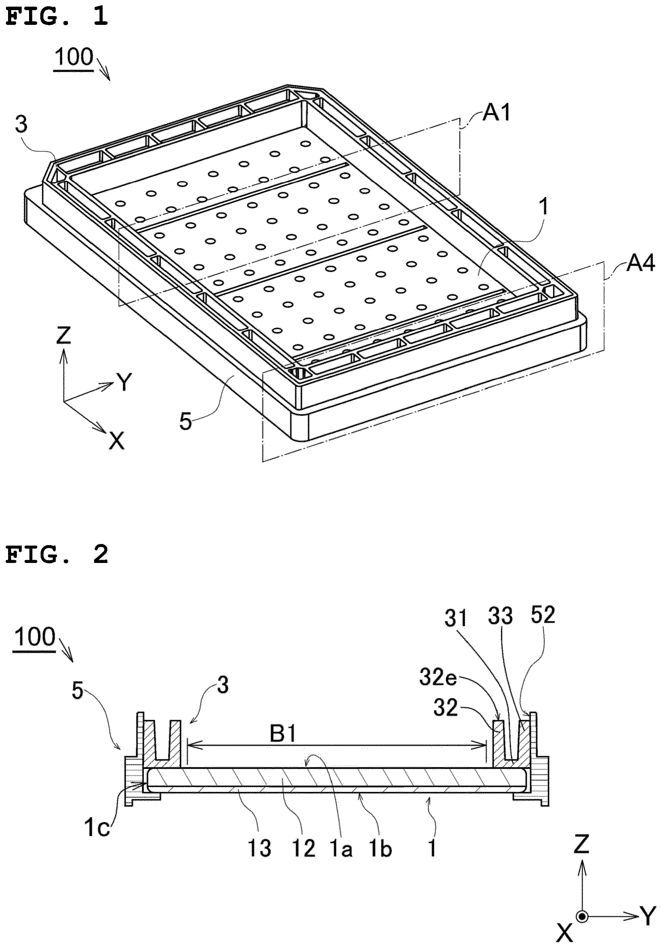

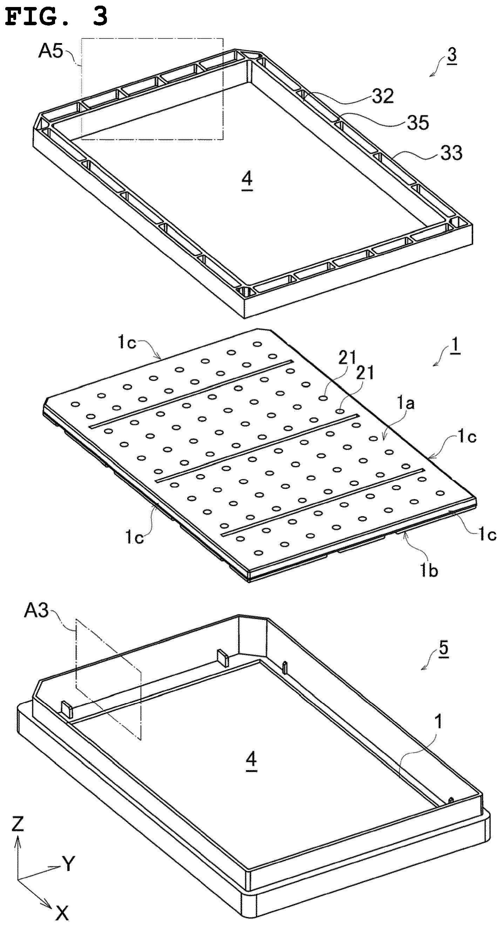

The first embodiment of the microfluidic device is described below. FIG. 1 is a schematic perspective view of a microfluidic device 100. FIG. 2 is a schematic cross-sectional view of the microfluidic device taken along plane A1 (parallel to YZ plane) in FIG. 1. FIG. 3 is a schematic exploded perspective view of the microfluidic device 100. The microfluidic device 100 includes a plate 1 for culturing and analyzing cells or tissues, a reservoir 3, and a holder 5 that supports the plate 1 from below. With reference to FIGS. 2 and 3, the microfluidic device 100 is formed by fitting the plate 1 inside the holder 5 from the upper side (+Z side) of the holder 5, and fitting the reservoir 3 inside the holder 5 from the upper side of the fitted plate 1.

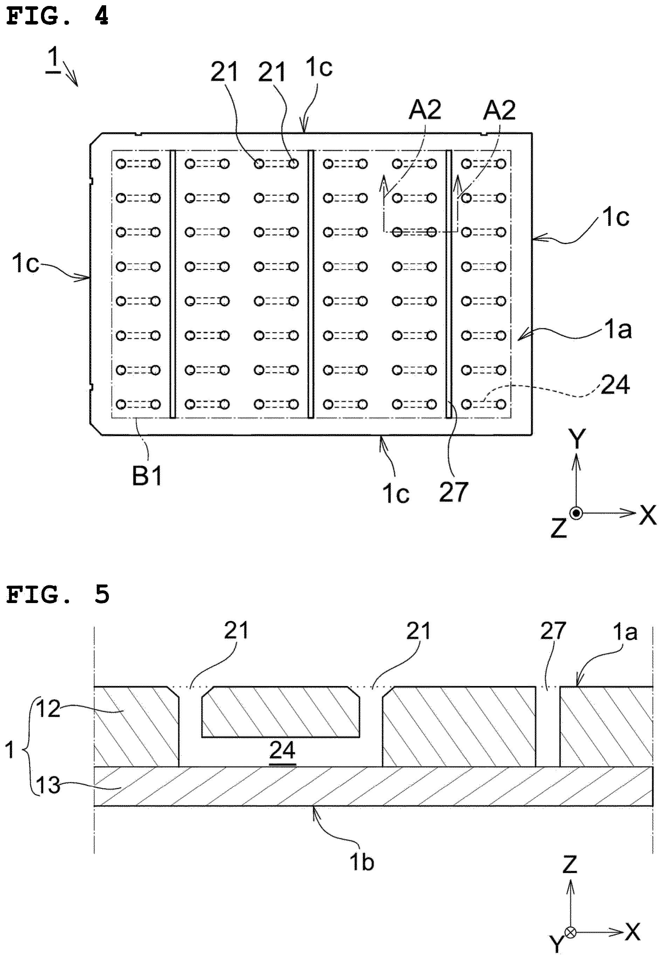

[Plate] The plate 1 is described below. FIG. 4 shows a top view of the plate 1. The plate 1 has a rectangular shape with its sides extending in the X direction and the Y direction. FIG. 5 is a schematic cross-sectional view of the plate taken along line A2 - A2 in FIG. 4. With reference to FIGS. 4 and 5, the plate 1 has a first main surface 1a with an opening area B1 including a plurality of openings 21 and slit-shaped openings 27, the openings for injecting fluid containing cells and tissues, a second main surface 1b being the opposite side of the first main surface 1a, and outer side surfaces 1c communicating the first main surface 1a and the second main surface 1b. The plate 1 is composed of the first substrate 12 and the second substrate 13, which are bonded together. The opening 21 is provided on a surface of the first substrate 12, the surface being not bonded to the second substrate 13. The surface of the first substrate 12 that is bonded to the second substrate 13 has grooves 24 for culturing and analyzing cells and tissues. In this embodiment, one groove 24 is connected to two openings 21.

The second substrate 13 is bonded to the first substrate 12 so as to cover the groove 24 connected to the two openings 21. The groove 24 serves as a hollow-shaped channel sandwiched between the two substrates (12, 13) by bonding the main surfaces of the first substrate 12 and the second substrate 13. This hollow-shaped channel is a microfluidic channel having a width and a depth on the order of micrometers. In the microfluidic channel, a small volume of fluid can be injected into the two openings 21 connected thereto and can be discharged from the microfluidic channel via the openings 21. The microfluidic channels are used as culture media or analysis sites for cells and tissues. The microfluidic channel may not necessarily be in a state where the fluid concerned flows through the channel; for example, the microfluidic channel may be in a state where the fluid has no flow, such as a state where fluid containing cells is stored in order to culture cells or tissues.

The bonding of the first substrate 12 and the second substrate 13 is performed, for example, by irradiating the bonding surfaces of the first substrate 12 and the second substrate 13 with ultraviolet light of 172 nm to activate the bonding surfaces and bond them together. In this embodiment, the thickness (dimension in the Z direction) of the second substrate 13 is thinner than the thickness (dimension in the Z direction) of the first substrate 12. The second substrate 13 is the same size as the first substrate 12 or smaller than the first substrate 12 when viewed from the +Z direction.

Resin materials constituting the first substrate 12 or the second substrate 13 include, for example, polymethyl methacrylate (PMMA), polycarbonate (PC), cycloolefin copolymer (COC), cycloolefin polymer (COP), polystyrene (PS), and silicone. In this embodiment, the first substrate 12 and the second substrate 13 are made of COP, which has high heat resistance and transparency and low autofluorescence in the fluorescence detection for analysis. The first substrate 12 and the second substrate 13 can be made from a combination of two or more of the resin materials described above. The first substrate 12 can be made of a material different from that of the second substrate 13.

The plate 1 can be any shape and may not be rectangular. The plate 1 may have a thickness (dimension in the Z direction) of 10 mm or less, preferably 7 mm or less, and more preferably 5 mm or less. The plate 1 may be composed of three or more substrates bonded together, or may be composed of a single substrate.

[Holder] With reference to FIG. 3, the holder in the present embodiment is described. In this embodiment, the holder 5 is a frame shape with a central area 4 therethrough. Since the central area 4 of the holder 5 goes through, light is irradiated from the upper side (+Z side) of the microfluidic device 100, then the light transmitted through the groove 24 of the plate 1 at the lower side (-Z side) of the microfluidic device 100 can be analyzed. Light may be irradiated from the lower side of the microfluidic device 100 and the light reflected or scattered inside the groove 24 of the plate 1 at the lower side of the microfluidic device 100 may be analyzed.

The holder 5 can also be a shape in which the central area 4 of the holder 5 does not go through, i.e., a shape with a bottom. In a case where the holder 5 is a shape with a bottom and unlikely to transmit light, light is irradiated from the upper side of the microfluidic device 100 and the light reflected or scattered inside the groove 24 of the plate 1 is received at the upper side of the holder 5 for analysis.

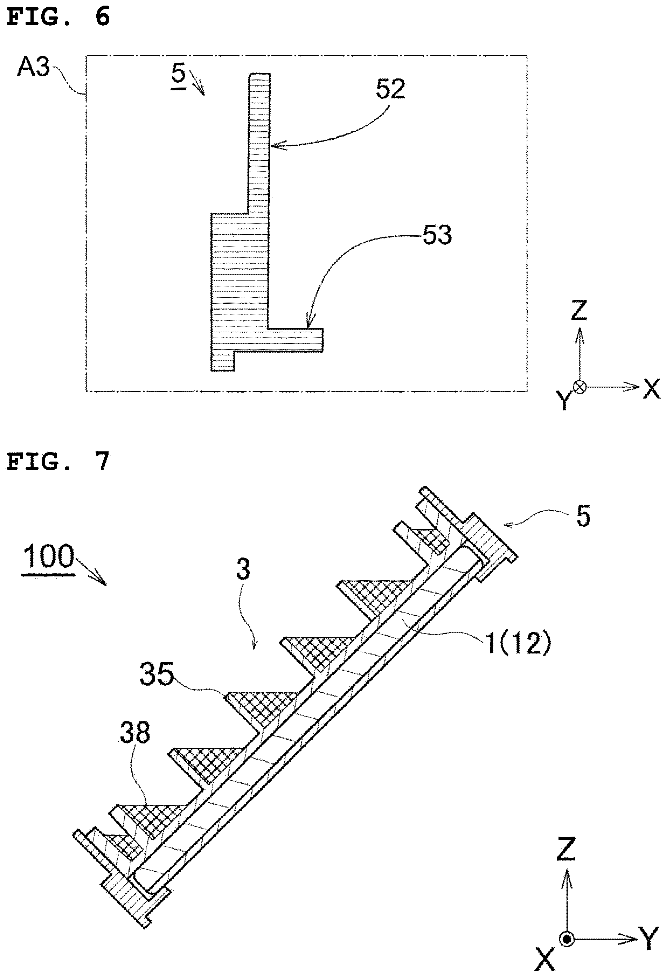

FIG. 6 is a schematic cross-sectional view of a holder 5 taken along the plane A3 (parallel to the XZ plane) in FIG. 3. With reference to FIGS. 2 and 6, the holder 5 has an inner wall surface 52 located outside the outer side surface 1c of the plate 1 on the XY plane, and a support surface 53 on which the second main surface 1b of the plate 1 is mounted. The inner wall surface 52 is positioned to face the outer side surface 1c of the plate 1. The support surface 53 is in contact with the second main surface 1b of the plate 1. In the present embodiment, the holder 5 is formed to surround the outer side surfaces 1c of the plate 1. The inner wall surface 52 surrounds all the outer side surfaces 1c of the plate 1, and the support surface 53 supports the area of the second main surface 1b except the opening area B1 of the plate 1.

The material of the holder 5 may include, for example, polymethyl methacrylate (PMMA), polycarbonate (PC), cycloolefin copolymer (COC), cycloolefin polymer (COP), polystyrene (PS), silicone, and other resin materials, or metal materials. In this embodiment, PS is used for the holder 5.

[Reservoir] With reference to FIGS. 2 and 3, the reservoir 3 is described. The reservoir 3 is a container having an opening with a bottom for being capable of storing liquid. When liquid containing cells or tissues is injected into the plate 1 for cultivation, analysis, or storage, the liquid injected into the plate 1 needs to be suppressed from evaporating. To suppress the evaporation of the liquid, the reservoir 3 is filled with liquid to maintain high humidity in the atmosphere of the plate 1. The evaporation of the liquid stored in the reservoir 3 can suppress the decrease in humidity of the atmosphere of the plate 1.

Here, even if a reservoir is provided inside the plate 1, the reservoir will not be deeper than the thickness of the plate 1. However, the reservoir 3 provided outside the plate 1 according to the present invention, allows the reservoir to be deeper than the thickness of the plate 1. The reservoir 3 provided outside the plate 1 of the present invention enables to increase the amount of liquid available for storage, compared to the case where the reservoir is provided inside the plate 1. Hence, since sufficient amount of liquid can be secured, the atmosphere of plate 1 can maintain high humidity. Also the liquid stored in the reservoir 3 needs to be replenished periodically when the liquid gradually decreases for evaporation. Since the reservoir 3 of the present invention has a larger volume than the reservoir provided inside the plate 1, the frequency of replenishment can be reduced to, for example, once every one to three days. The reservoir of the present embodiment can store a total volume of 3 ml (milliliters) or more.

The reservoir 3 of the present embodiment is a frame shape with going through the central area 4 (see FIG. 3). The reservoir 3 includes an opening with a bottom, the opening standing above the first main surface 1a of the plate 1. The reservoir 3 includes a bottom 31, an inner frame 32 and an outer frame 33, those of the frames standing upward (+Z direction) from the bottom 31 (see FIG. 2). The bottom 31 is located outside the opening area B1 of the plate 1. The inner frame 32 is separated from the inner wall surface 52 of the holder 5, and located inside the inner wall surface 52. The outer frame 33 is located between the inner wall surface 52 and the inner frame 32. In this embodiment, the outer frame 33 is in contact with at least part of the inner wall surface 52. The reservoir 3 is surrounded by the bottom 31, the inner frame 32, and the outer frame 33 to form a cylinder-shaped space, and is capable of storing liquid in this space.

Water, for example, is used as the liquid to be stored in the reservoir 3. Chemicals such as antibiotics may be added to the liquid stored in the reservoir 3 to prevent the growth of mold and bacteria in the culture medium.

The reservoir 3 has a larger volume with its deeper depth. Here, the upper end 32e of the inner frame 32 is higher (on the +Z side) than the first main surface 1a of the plate 1 (see FIG. 2). This configuration indicates that a reservoir is allowed to be designed deeper compared with a reservoir provided inside the plate 1 to increase the amount of liquid that can be stored.

As shown in FIGS. 1 and 3, the reservoir 3 in this embodiment is configured separately from the holder 5. The microfluidic device 100 consists of the plate 1 fitted into the holder 5, and the reservoir 3 fitted into the holder 5, as described above. Here, the bottom 31 of the fitted reservoir 3 is located above the first main surface 1a, covering the outer periphery of the plate 1 (the area outside the opening area B1) (see FIG. 2). Since the reservoir 3 can be stacked on the plate 1, the reservoir 3 can have a larger area of the bottom 31 compared with a reservoir provided outside on the same surface as the plate 1. Hence, the reservoir 3 can store an increased amount of liquid. The reservoir 3 itself is designed in a size and a shape that does not cover the opening area B1, and allows liquid to be injected into the opening 21 while the reservoir 3 is fitted into the holder 5.

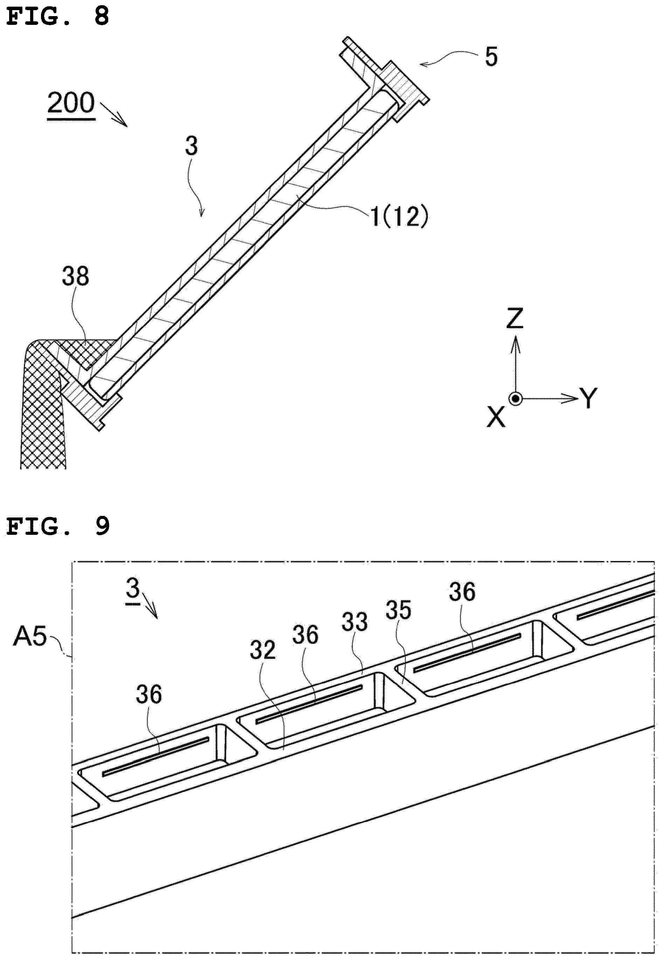

The reservoir 3 of this embodiment is partitioned into a plurality of cells by partitioning plates 35 that communicate between the inner frame 32 and the outer frame 33 (see FIG. 3). In this embodiment, the reservoir 3 has 24 cells; however, the number of cells can be any number. With reference to FIGS. 7 and 8, the effect of partitioning the reservoir 3 into a plurality of cells is explained. FIG. 7 corresponds to a cross-sectional view of the microfluidic device 100 taken along plane A4 (parallel to the YZ plane) in FIG. 1 at a predetermined tilted angle (in this case 45 degrees around the X axis). Fig. 7 illustrates the reservoir 3 in a state in which liquid 38 is injected into each cell of the reservoir 3 of the microfluidic device 100.

The microfluidic device 100 may be tilted for analysis or observation of cells or tissues contained in the microfluidic device 100 (especially in the groove 24). Moreover, the microfluidic device 100 may be unintentionally or inevitably tilted during transportation of the microfluidic device 100. FIG. 7 is a drawing that reflects this situation.

In contrast to the drawing in FIG. 7, FIG. 8 is a cross-sectional view of the microfluidic device 200 in which the reservoir 3 is not partitioned into a plurality of cells. As shown in FIG. 8, when the microfluidic device 200 is tilted at a predetermined angle (in this case, 45 degrees around the X-axis), the liquid 38 accumulates in the lower part of the reservoir 3, and the liquid 38 overflows and flows out of the reservoir 3.

On the other hand, in the case of the reservoir 3 partitioned into each cell by the partitioning plates 35 (see FIG. 7), even if the microfluidic device 100 is tilted, the liquid 38 is not likely to flow out over each cell. In other words, in the case that the microfluidic device 100 is tilted, the configuration shown in FIG. 7 is more likely to prevent the liquid from flowing out of the reservoir 3 compared with the configuration shown in FIG. 8.

As shown above, the partitioning plates 35 provided in the reservoir have the effect of preventing the liquid in the reservoir 3 from flowing out, even if the microfluidic device 100 is tilted at a certain angle. Moreover, in the case of transporting the microfluidic device 100, the reservoir 3 provided with the partitioning plates 35 prevents the liquid in the reservoir 3 from flowing out. The partitioning plates 35 may be integrally formed with the bottom 31, then inner frame 32, and the outer frame 33, or they may be configured to be retrofitted.

The reservoir 3 may be provided with a marker that serves as an indicator of the amount of replenishment liquid in order to inject the appropriate amount of liquid into the reservoir 3. FIG. 9 is an enlarged view of the A5 area in FIG. 3, with an example of the markers added. The reservoir 3 is provided with protrusions 36 on the inner surface of the outer frame 33 that indicates the maximum liquid level height as an indicator. The liquid is replenished in the reservoir 3 until the liquid level reaches the position of the protrusion 36 by an operator replenishing the liquid or a replenishment device.

The protrusion 36 can be any shape. The marker may be a concave section, a printed mark, or any marker other than the projection 36. The markers may be provided on the outer surface of the outer frame 33 or on the inner frame 32. The markers may be located in all the cells or only in part of the cells. Each marker may have a different height position at each cell that constitutes the reservoir 3 to set the appropriate amount of liquid for each cell.

The materials of the bottom 31, the inner frame 32, the outer frame 33, and the partitioning plate 35 of the reservoir 3 include, for example, polymethyl methacrylate (PMMA), polycarbonate (PC), cycloolefin copolymer (COC), cycloolefin polymer (COP), polystyrene (PS), silicone, and other resin materials, or metal materials. In this embodiment, PS is used for the reservoir 3. In at least part of the reservoir 3, an evaporation enhancing member (e.g., a hygroscopic gel or sponge liquid-absorbing material, or an evaporation mechanism that increases the area of the gas-liquid interface, such as fins) may be mounted inside the reservoir 3. Mounting the evaporation enhancing member in the reservoir 3 allows the atmosphere to maintain at a high humidity level, thus effectively suppressing the evaporation of the liquid from the plate 1.

Incidentally, the degree of evaporation of the liquid from the plate 1 and the reservoir 3 correlates with the area of the gas-liquid interface. In the case of a plate 1 having a large number of the openings 21 or a large area of the openings 21 per each opening, the plate 1 has a large area of the openings 21 in total. The increased total area of the openings 21 allows the area of the gas-liquid interface at the openings 21 to increase, thus causing the liquid to evaporate more quickly. The similar manner applies to the reservoir 3; the increased area of the reservoir allows the gas-liquid interface in the reservoir 3 to increase, thus causing the liquid to evaporate more quickly.

To suppress the evaporation of the liquid from the openings 21, the area of the gas-liquid interface in the reservoir 3 may be designed to a size corresponding to the total area of the openings 21. The amount of area of the gas-liquid interface in the reservoir 3 varies considerably depending on the size of the inner bottom surface of the reservoir 3. Hence, the total area of the inner bottom surface of the reservoir 3 may be preferably at least twice the total area of the openings 21, and may be more preferably 10 times or more. Setting this ratio (i.e. ratio of the total area of the inner bottom surface of the reservoir 3 to the total area of the openings 21) being twice or larger or even 10 times or larger allows the amount of evaporation from the entire reservoir 3 to become larger than the amount of evaporation from the entire openings 21, thereby suppressing the evaporation of liquid from the openings 21. A heat source or a heat conductive member connected with an external heat source may be mounted in the vicinity of the reservoir 3 in order to control the amount of evaporation from the reservoir 3.

When the microfluidic device 100 is stored in a storage chamber (also called an incubator) that is configured to have a high humidity environment (e.g., humidity of 95% or higher), the water vapor in the storage chamber provides atmosphere of the microfluidic device 100, thus easily controlling the humidity of the atmosphere of the microfluidic device 100. This configuration further suppresses the evaporation of liquid components from the openings 21 of the microfluidic device 100. Note that the reservoir 3 serves to control the humidity of the atmosphere of the plate 1 even when the plate 1 is stored in the storage chamber, since the storage chamber does not always strictly maintain a constant humidity level due to the opening and closing operation of its door or the like.

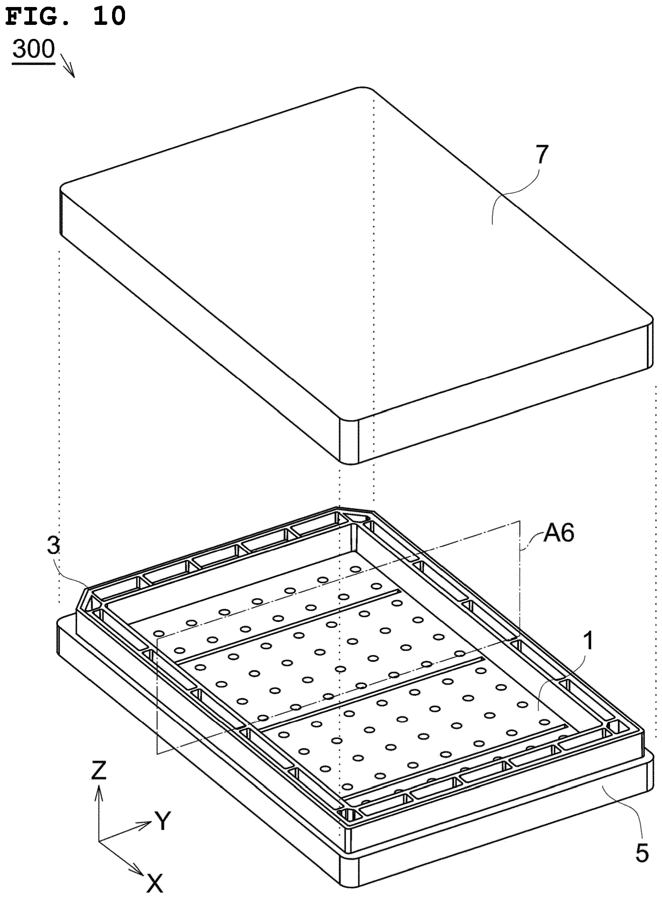

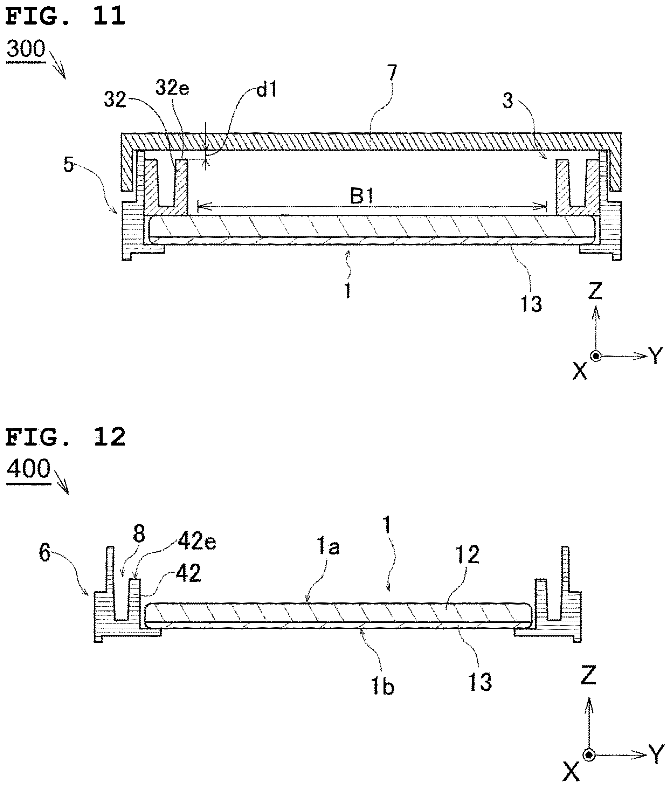

With reference to FIGS. 10 and 11, modified examples of a microfluidic device are shown. FIG. 10 is a schematic perspective view of the microfluidic device 300. The microfluidic device 300 includes a plate 1, a reservoir 3, a holder 5, and a cover 7. In FIG. 10, it is shown that the plate 1 and the reservoir 3 are fitted into the holder 5, and the cover 7 is separated from the holder 5. FIG. 11 is a cross-sectional view taken along the plane A6 (parallel to the YZ plane) in FIG. 10. In FIG. 11, the cover 7 is shown attached to the holder 5.

As shown in FIG. 11, the cover 7 is attached to the holder 5 to cover the opening area B1 and the reservoir 3. This allows the atmosphere in the opening area B1 to be limited in a small space, thereby enabling better control of humidity therein. A gap d1 is formed between the cover 7 and the upper end 32e of the inner frame 32 of the reservoir 3. This configuration allows the liquid stored in the reservoir 3 to evaporate and flow into the opening area B1 even with the cover 7 attached to the holder 5.

In FIG. 11, a small gap (not shown) that is capable of ventilation is provided between the cover 7 and the holder 5. Since the inside and the outside of the microfluidic device 300 are configured to be ventilated, the high-humidity atmosphere in the storage chamber can flow into the microfluidic device 300 even with the cover 7 attached to the holder 5. In the microfluidic device 300 configured to be ventilated, gas flowing from the outside to the inside passes near the liquid surface of the reservoir 3, thus enhancing the evaporation of the liquid in the reservoir 3. In addition, the small gap between the cover 7 and the holder 5 may also serve as an inlet for carbon dioxide (CO2), which may be added with its concentration higher than that of the atmosphere in order to maintain the acidity of the liquid injected into the microfluidic device.

The cover 7 may be composed of a light transmissive material, which enables the plate 1 to be analyzed with the cover 7 covering thereon. The cover 7 may also be provided with a port that allows liquid to be injected into the reservoir 3.

### <Second Embodiment>

With reference to FIG. 12, asecond embodiment of a microfluidic device is described. The microfluidic device 400 shown in FIG. 12 has a reservoir 8 including a holder 6. In other words, the holder 6 is integrated into the reservoir 8. In this embodiment, the upper end 42e of the inner frame 42 is also higher than the first main surface 1a of the plate 1; that is, the opening with the bottom stands above the first main surface.

### INDUSTRIAL APPLICABILITY

Cultivation and analysis of cells and tissues is described above as applications of microfluidic devices. However, the microfluidic devices of the present invention can be applied to applications other than the cultivation and the analysis of cells and tissues. For example, the microfluidic devices of the present invention can be used for mixing, separation, reaction, synthesis, extraction, or analysis of chemicals.

### REFERENCE SIGNS LIST

- 1: plate
- 1a: first main surface of plate
- 1b: second main surface of plate
- 1c: outer side surface of plate
- 3, 8: reservoir
- 4: central area
- 5, 6: holder
- 7: cover
- 12: first substrate
- 13: second substrate
- 21: opening
- 24: groove
- 27: slit-shaped opening
- 31: bottom
- 32, 42: inner frame
- 33: outer frame
- 35: partitioning plate
- 38: liquid
- 52: inner wall surface
- 53: support surface
- 100, 200, 300, 400: microfluidic device

## Claims

1. A microfluidic device (100) comprising:
a plate (1) having a first main surface (1a) that has an opening area in which a plurality of openings (21) for injecting fluid are formed, a second main surface (1b) being the opposite side of the first main surface (1a), and an outer side surface (1c) communicating the first main surface (1a) and the second main surface (1b); wherein the plate (1) is composed of a first substrate (12) and a second substrate (13) which are bonded together so that grooves (24) serving as hollow-shaped channels are sandwiched between the two substrates (12, 13) and are connected to two openings (21),
wherein the microfluidic device (100) further comprises
a reservoir (3) that has at least one opening with a bottom and is capable of storing liquid (38), and
a holder (5) having an inner wall surface (52) that is located outside the outer side surface (1c) of the plate (1) and a support surface (53) on which the second main surface (1b) of the plate (1) is mounted,
**characterized in that**
the at least one opening of the reservoir (3) stands above the first main surface (1a) outside the opening area.

2. The microfluidic device (100) according to claim 1, wherein the reservoir (3) includes a bottom (31) located outside the opening area of the plate (1), an inner frame (32) standing upward from the bottom (31) to above the first main surface (1a) and being separated inward from the inner wall surface (52) of the holder (5), and an outer frame (33) standing upward from the bottom (31) between the inner wall surface (52) and the inner frame (32).

3. The microfluidic device (100) according to claim 2, wherein the reservoir (3) is configured separately from the holder (5), and the bottom (31) is located above the first main surface (1a).

4. The microfluidic device (100) according to claim 2 or 3, wherein the reservoir (3) is partitioned into a plurality of cells by a partitioning plate (35) that communicates between the inner frame (32) and the outer frame (33).

5. The microfluidic device (100) according to any one of claims 1 to 4, wherein the total area of the inner bottom surface of the reservoir (3) is more than twice the total area of the openings in the plate (1).

6. The microfluidic device (100) according to any one of claims 1 to 5, wherein the inner side surface of the reservoir (3) has a marker that serves as an indicator of the amount of replenishment fluid.

7. The microfluidic device (100) according to any one of claims 1 to 6, wherein at least a portion of the reservoir (3) is provided with an evaporation enhancing member increasing the area of the gas-liquid interface.

8. The microfluidic device (100) according to any one of claims 1 to 7, wherein the microfluidic device (100) is provided with a cover (7) that covers the opening area and the reservoir (3) to form a gap between the cover (7) and the upper end of the reservoir (3).

9. The microfluidic device (100) according to any one of claims 1 to 8, wherein the plate (1) is constituted by bonding at least two substrates (12, 13) and includes a space between the bonded substrates (12, 13) for being capable of culturing cells or tissues.

## Patentansprüche

1. Mikrofluidische Vorrichtung (100), umfassend:
eine Platte (1) mit einer ersten Hauptoberfläche (1a), die einen Öffnungsbereich aufweist, in dem eine Vielzahl von Öffnungen (21) zum Einspritzen von Fluid ausgebildet sind, einerzweiten Hauptoberfläche (1b), die die gegenüberliegende Seite der ersten Hauptoberfläche (1a) ist, und einer äußeren Seitenfläche (1c), die die erste Hauptoberfläche (1a) und die zweite Hauptoberfläche (1b) verbindet, wobei die Platte (1) aus einem ersten Substrat (12) und einem zweiten Substrat (13) zusammengesetzt ist, die so miteinander verbunden sind, dass Nuten (24), die als hohlförmige Kanäle dienen, zwischen den beiden Substraten (12, 13) eingeschlossen sind und mit zwei Öffnungen (21) verbunden sind,
wobei die mikrofluidische Vorrichtung (100) weiter umfasst:
ein Reservoir (3), das mindestens eine Öffnung mit einem Boden aufweist und Flüssigkeit (38) speichern kann, und
einen Halter (5) mit einer inneren Wandfläche (52), die sich außerhalb der äußeren Seitenfläche (1c) der Platte (1) befindet, und einer Stützfläche (53), auf der die zweite Hauptfläche (1b) der Platte (1) angebracht ist,
**dadurch gekennzeichnet, dass**
die mindestens eine Öffnung des Behälters (3) oberhalb der ersten Hauptfläche (1a) außerhalb des Öffnungsbereichs steht.

2. Mikrofluidische Vorrichtung (100) nach Anspruch 1, wobei das Reservoir (3) einen Boden (31) umfasst, der sich außerhalb des Öffnungsbereichs der Platte (1) befindet, einen inneren Rahmen (32), der vom Boden (31) nach oben bis über die erste Hauptoberfläche (1a) steht und nach innen von der inneren Wandfläche (52) des Halters (5) getrennt ist, und einen äußeren Rahmen (33), der vom Boden (31) zwischen der inneren Wandfläche (52) und dem inneren Rahmen (32) nach oben steht.

3. Mikrofluidische Vorrichtung (100) nach Anspruch 2, wobei das Reservoir (3) getrennt vom Halter (5) ausgebildet und der Boden (31) oberhalb der ersten Hauptoberfläche (1a) angeordnet ist.

4. Mikrofluidische Vorrichtung (100) nach Anspruch 2 oder 3, wobei das Reservoir (3) durch eine Trennplatte (35), die zwischen dem inneren Rahmen (32) und dem äußeren Rahmen (33) angeordnet ist, in eine Vielzahl von Zellen unterteilt ist.

5. Mikrofluidische Vorrichtung (100) nach einem der Ansprüche 1 bis 4, wobei die Gesamtfläche der inneren Bodenfläche des Reservoirs (3) mehr als doppelt so groß ist wie die Gesamtfläche der Öffnungen in der Platte (1).

6. Mikrofluidische Vorrichtung (100) nach einem der Ansprüche 1 bis 5, wobei die innere Seitenfläche des Reservoirs (3) eine Markierung aufweist, die als Indikator für die Menge der Nachfüllflüssigkeit dient.

7. Mikrofluidische Vorrichtung (100) nach einem der Ansprüche 1 bis 6, wobei mindestens ein Teil des Reservoirs (3) mit einem verdampfungsfördernden Element versehen ist, das die Fläche der Gas-Flüssigkeits-Grenzfläche vergrößert.

8. Mikrofluidische Vorrichtung (100) nach einem der Ansprüche 1 bis 7, wobei die mikrofluidische Vorrichtung (100) mit einer Abdeckung (7) versehen ist, die den Öffnungsbereich und das Reservoir (3) abdeckt, um einen Spalt zwischen der Abdeckung (7) und dem oberen Ende des Reservoirs (3) zu bilden.

9. Mikrofluidische Vorrichtung (100) nach einem der Ansprüche 1 bis 8, wobei die Platte (1) durch Verbinden von mindestens zwei Substraten (12, 13) gebildet ist und einen Raum zwischen den verbundenen Substraten (12, 13) enthält, um Zellen oder Gewebe kultivieren zu können.

## Revendications

1. Dispositif microfluidique (100) comprenant :
une plaque (1) ayant une première surface principale (1a) qui possède une zone d'ouvertures dans laquelle sont formées une pluralité d'ouvertures (21) pour l'injection de fluide, une seconde surface principale (1b) qui est le côté opposé de la première surface principale (1a), et une surface latérale extérieure (1c) communiquant avec la première surface principale (1a) et la seconde surface principale (1b) ; dans lequel la plaque (1) est composée d'un premier substrat (12) et d'un second substrat (13) qui sont liés ensemble de sorte que des rainures (24) servant de canaux en forme de creux sont prises en sandwich entre les deux substrats (12, 13) et sont reliées à deux ouvertures (21), dans lequel le dispositif microfluidique (100) comprend en outre
un réservoir (3) qui possède au moins une ouverture avec un fond et qui peut stocker du liquide (38), et un support (5) possédant une surface de paroi intérieure (52) qui est située à l'extérieur de la surface latérale extérieure (1c) de la plaque (1) et une surface de support (53) sur laquelle est montée la seconde surface principale (1b) de la plaque (1),
**caractérisé en ce que**
l'au moins une ouverture du réservoir (3) se trouve au-dessus de la première surface principale (1a) à l'extérieur de la zone d'ouvertures.

2. Dispositif microfluidique (100) selon la revendication 1, dans lequel le réservoir (3) comporte un fond (31) situé à l'extérieur de la zone d'ouvertures de la plaque (1), un cadre intérieur (32) s'élevant vers le haut à partir du fond (31) jusqu'au-dessus de la première surface principale (1a) et étant séparé vers l'intérieur de la surface de paroi intérieure (52) du support (5), et un cadre extérieur (33) s'élevant vers le haut à partir du fond (31) entre la surface de paroi intérieure (52) et le cadre intérieur (32) .

3. Dispositif microfluidique (100) selon la revendication 2, dans lequel le réservoir (3) est configuré séparément du support (5), et le fond (31) est situé au-dessus de la première surface principale (1a).

4. Dispositif microfluidique (100) selon la revendication 2 ou 3, dans lequel le réservoir (3) est divisé en une pluralité de cellules par une plaque de séparation (35) qui communique entre le cadre intérieur (32) et le cadre extérieur (33).

5. Dispositif microfluidique (100) selon l'une quelconque des revendications 1 à 4, dans lequel la superficie totale de la surface inférieure interne du réservoir (3) est supérieure à deux fois la superficie totale des ouvertures dans la plaque (1).

6. Dispositif microfluidique (100) selon l'une des revendications 1 à 5, dans lequel la surface latérale intérieure du réservoir (3) comporte un marqueur qui sert d'indicateur de la quantité de fluide de réapprovisionnement.

7. Dispositif microfluidique (100) selon l'une des revendications 1 à 6, dans lequel au moins une partie du réservoir (3) est pourvue d'un élément favorisant l'évaporation augmentant la surface de l'interface gaz-liquide.

8. Dispositif microfluidique (100) selon l'une quelconque des revendications 1 à 7, dans lequel le dispositif microfluidique (100) est pourvu d'un couvercle (7) qui recouvre la zone d'ouvertures et le réservoir (3) pour former un espace entre le couvercle (7) et l'extrémité supérieure du réservoir (3).

9. Dispositif microfluidique (100) selon l'une quelconque des revendications 1 à 8, dans lequel la plaque (1) est constituée par la liaison d'au moins deux substrats (12, 13) et comprend un espace entre les substrats liés (12, 13) pour pouvoir cultiver des cellules ou des tissus.
